Europäisches Patentamt

European Patent Office

Office européen des brevets

(11) Veröffentlichungsnummer: **0 282 696 B1**

# (12) EUROPÄISCHE PATENTSCHRIFT

(45) Veröffentlichungstag der Patentschrift: **11.11.92**

(51) Int. Cl.5: **A61K 31/44**, A61K 31/66

(21) Anmeldenummer: **88100747.0**

(22) Anmeldetag: **20.01.88**

Die Akte enthält technische Angaben, die nach dem Eingang der Anmeldung eingereicht wurden und die nicht in dieser Patentschrift enthalten sind.

(54) **Verwendung von Pyridoxin-Derivaten bei der Prophylaxe und Therapie von Hyperlipidämien und Atherosklerose.**

(30) Priorität: **18.02.87 DE 3705549**

(43) Veröffentlichungstag der Anmeldung:
**21.09.88 Patentblatt 88/38**

(45) Bekanntmachung des Hinweises auf die Patenterteilung:
**11.11.92 Patentblatt 92/46**

(84) Benannte Vertragsstaaten:
**AT BE CH DE ES FR GB GR IT LI LU NL SE**

(56) Entgegenhaltungen:
DE-A- 1 915 497
FR-A- 2 295 741
FR-A- 2 349 330
FR-M- 6 707
GB-A- 1 070 120

CHEMICAL ABSTRACTS, Band 79, 1973 Seite 392, Zusammenfassung 126314e, Columbus, 0hio, US

REV. BRAS. MED, Band 29, Nr. 1, Januar 1972, Seiten 38-41; P. SCHLESINGER

CHEMICAL ABSTRACTS, Band 70, Nr.19 12.

Mai 1969, Seite 183, Zusammenfassung 86180p, Columbus, 0hio, US H. NAKAMURA et al.

Arzneimittelforschung Drug Research 36(II),8, 1210-1215 (1986)

"ROTE LISTE" 1988, 83-026, 83029-83032, 83137, 83140

The Merck Manual, Diagnosis and Therapy, 14 Aufl. 1982, Seiten 386-389

(73) Patentinhaber: **STEIGERWALD ARZNEIMITTEL-WERK GMBH**
**Havel Strasse 5**
**W-6100 Darmstadt(DE)**

(72) Erfinder: **Speck, Ulrich, Dr.**
**Benediktiner Strasse 50**
**W-1000 Berlin 28(DE)**

(74) Vertreter: **Wablat, Wolfgang**
**Patentanwalt Dr.-Ing. Dr. jur. Dipl.-Chem. W. Wablat Potsdamer Chaussee 47**
**W-1000 Berlin 38(DE)**

EP 0 282 696 B1

**Beschreibung**

Die Erfindung betrifft die Verwendung von ausgewählten Pyridoxin-Derivaten zur Herstellung eines Arzneimittels für die Prophylaxe und Therapie von Hyperlipidämien und Atherosklerose. Ausgenommen sind insbesondere Pyridoxin-5'-phosphorsäureester-glutaminat und -asparaginat, die in der DE 24 61 742 C2 beschrieben sind.

Atherosklerose zählt zu den häufigsten Erkrankungen und Todesursachen (Atherosklerose der Koronarien) in den entwickelten Ländern. Atherosklerose ist ein sich meist langsam entwickelnder Prozeß, der komplexe Veränderungen der Struktur und Funktion der Blutgefäße umfaßt, bis schließlich klinisch deutlich sichtbare Erkrankungen, wie Angina pectoris und Herzinfarkt durch Verengung und Verschluß von Herzkranzgefäßen, eine Minderdurchblutung der Extremitäten oder der Hirninfarkt auftreten. Lange vor dem dramatischen Endstadium der Erkrankung kommt es häufig zu jahrelangen starken Beschwerden und zum Siechtum. Die Behandlungsmöglichkeiten in fortgeschrittenem Stadium sind sehr begrenzt, aufwendig und invasiv, wie z.B. die Operation am offenen Herzen oder die Amputation von Extremitäten.

Abgesehen von relativ seltenen Fällen schwerer erblicher Stoffwechselentgleisungen ist die Atherosklerose zu einem bedeutenden Anteil eine Zivilisationskrankheit. Das Atheroskleroserisiko wird insbesondere durch Rauchen (Nikotin) erhöht. Weitere Risikofaktoren sind beispielsweise Bluthochdruck, mangelnde Bewegung, Diabetis mellitus und Niereninsuffizienz.

Von herausragender Bedeutung für die Prophylaxe der Atherosklerose war die Erkenntnis des Zusammenhanges zwischen erhöhten Blutfettwerten und einem erhöhten Atheroskleroserisiko bis zu einem erhöhten Risiko des Todes durch Herzinfarkt (vgl. Dayton, S., Chapman J., Pearce M., Popiak G.,Cholesterol, atherosclerosis, ischaemic heart disease, and stroke, Am.J. Med. 72, 97 (1970)).

Cholesterinreiche Nahrung und ein hoher Anteil von tierischen Fetten (gesättigten Fettsäuren) in der Nahrung erhöhen den Gehalt des Blutes an Lipoproteinen in unerwünschter Weise und führen damit zu einer beschleunigten Entwicklung atherosklerotischer Läsionen.

Da in den atheromatösen Plaques hauptsächlich Cholesterin gefunden wurde, erscheint ein Zusammenhang zwischen erhöhtem Cholesterinspiegel und der Atherosklerose als gesichert.

Zum Transport der wasserunlöslichen Lipide im Blut dienen Lipoproteine. Dabei wird zwischen LDL-(low density lipoproteins) und HDL- (high density lipoproteins)-Lipoproteinen unterschieden. In der Atherogenese ist das LDL-Cholesterin von entscheidender Bedeutung. Das HDL-Cholesterin dagegen transportiert überschüssiges Cholesterin aus der Peripherie in die Leber zurück. Ihm wird eine eher protektive Wirkung zugerechnet.

Die Anreicherung von Cholesterin in den Mediazellen der Arterienwände hängt also nicht nur von der Konzentration des Gesamtcholesterins im Serum, sondern auch vom Verhältnis LDL zu HDL ab.

Unter dem Einfluß erhöhter Blutfettwerte, insbesondere erhöhter LDL-Konzentrationen im Blut, kommt es zu verstärkten Lipideinlagerungen in die Arterienwände, die ihrerseits den Ausgangspunkt für weitere atherosklerotische Veränderungen bilden oder diese jedenfalls verstärken.

Ziel der Atheroseprophylaxe wurde daher zunehmend eine Senkung der Blutfettwerte im allgemeinen und des LDL-Cholesterinwertes im besonderen.

Dieses Ziel kann auf unterschiedliche Weisen erreicht werden:
Ein Teil des Cholesterins und der ebenfalls unerwünschten gesättigten Fettsäuren werden mit der Nahrung zugeführt. Daher ist die Einhaltung einer Diät, die wenig Cholesterin und tierische Fette enthält, erste Voraussetzung für eine erfolgreiche Behandlung oder Prophylaxe der Atherosklerose.

Die Einhaltung einer Diät ist jedoch häufig schwierig und auch nicht ausreichend. Die atherogenen Lipide werden nämlich nicht nur mit der Nahrung aufgenommen, sondern auch im Körper gebildet. Um die Konzentrationen auch der endogenen Lipide im Blut zu reduzieren oder zumindest ihre atherogene Wirkung zu mindern, wurde in den vergangenen zwanzig Jahren eine große Zahl von Arzneistoffen entwickelt oder für diesen Zweck empfohlen.

Ein einfacher und relativ unschädlicher Ansatzpunkt für die Senkung der Cholesterinkonzentration im Blut ergibt sich aus dessen Ausscheidungsmechanismus. Cholesterin wird in der Leber in Gallensäuren umgewandelt und gelangt also solche in den Darm. Die Gallensäuren werden zu einem wesentlichen Teil rückresorbiert und wiederverwendet. Dieser Kreislauf läßt sich unterbrechen, wenn die Gallensäuren von nicht resorbierenden Substanzen im Darm festgehalten werden.

Solche Substanzen sind feste Ionenaustauscher, bestimmte Quellstoffe , wie beispielsweise Guar, aber auch übliche Nahrungsmittelbestandteile, wie beispielsweise Faserstoffe. Die Leber wird unter diesen Umständen veranlaßt, mehr Cholesterin in Gallensäuren umzuwandeln, womit dessen Ausscheidung beschleunigt wird . Nachteil dieses Therapiekonzeptes ist die außerordentlich hohe Dosierung der betreffenden Präparate (8 - 24 g/Tag) und die unbequeme Einnahme.

Weiterhin sind eine Reihe systemisch wirkender, sogenannter Lipidsenker in die Therapie eingeführt worden. Dazu gehören beispielsweise Derivate der Clofibrinsäure, welche die Synthese von Cholesterin in der Leber hemmen. Aufgrund der erheblichen Nebenwirkungen und der erforderlichen Therapiedauer werden Clofibrate nur bei schweren Fettstoffwechselstörungen eingesetzt (vgl. Scheffler,W. und Schwartzkopff, W., Frequently used lipid-lowering drugs have no guaranteed effect. Artery 8, 120 - 127 (1980)).

Aber auch andere synthetische und selbst natürliche (Nicotinsäurederivate) Arzneimittel verursachen zum Teil unvertretbare Nebenwirkungen oder müssen so hoch dosiert werden, daß sich bei einer Langzeittherapie insbesondere bei älteren oder nieren- bzw. leberkranken Patienten eine kaum vertretbare Stoffwechselbelastung ergibt.

Bestimmte Derivate des Pyridoxinphosphats sind in der DE- 24 61 742 C2 als lipidsenkende und antiatherosklerotische Wirkstoffe beschrieben. Dabei handelt es sich um Pyridoxin-5′-phosphorsäureester-glutaminate und -asparaginate. Ihre Wirkung wird auf die Kombination des Pyridoxinphosphats mit Glutaminsäure oder Asparaginsäure zurückgeführt. Trotz ihrer unstreitigen Wirksamkeit weisen die bekannten Substanzen auch eindeutige Nachteile auf. Es sind im engen Sinne keine natürlichen Heilmittel mehr, die Chemie dieser Substanzen ist wenig klar, ihre Stabilität ist schon bei Zusatz geringster Feuchtigkeitsmengen fragwürdig und in vivo, z.B. bei der Resorption sicher nicht gegeben; ferner muß die Dosierung relativ hoch gewählt werden. Aufgrund der Instabilität lassen sich die in der DE 24 61 742 C2 aufgeführten Wirkstoffe auch nicht in Form von Infusionen oder Trinkampullen oder andersartigen Lösungen verabfolgen. Gerade die Einnahme in flüssiger Form ist jedoch für die meist betroffenen älteren Patienten besonders angenehm.

Unter ungünstigen Nebenbedingungen können Einzelbestandteile der in der DE 24 61 742 C2 beschriebenen Substanzen sich bei einer Behandlung der betroffenen Patienten sogar ungünstig auswirken.

Es wäre also im Hinblick auf die in der Regel betroffenen älteren, vielfältig kranken Patienten und die Notwendigkeit der oft lebenslangen Einnahme lipidsenkender Medikamente sehr wünschenswert, einen natürlichen, gut verträglichen und niedrig dosierbaren Arzneistoff zur Verfügung zu haben. Weiterhin sollte dieser Arzneistoff chemisch eindeutig charakterisierbar, stabil gegenüber äußeren Einflüssen sowie nach oraler Gabe möglichst unverändert und vollständig resorbierbar sein. Der Arzneistoff sollte die Stoffwechsellage in einer Weise ändern, daß es unter sonst gleichen Bedingungen zu einer Verminderung der Einlagerung von Lipiden in die Arterienwand kommt und damit zu einer Verzögerung, Unterbrechung oder sogar Rückbildung atherosklerotischer Veränderungen.

Aufgabe der Erfindung ist es somit, ein neues Mittel zur Prophylaxe und Therapie von Hyperlipidämien und Atherosklerose mit den genannten Eigenschaften zur Verfügung zu stellen. Dieses soll auch zur Behandlung von Personen, die trotz Diät erhöhte Blutfettkonzentrationen aufweisen, dienen.

Bei der Suche nach einem geeigneten Wirkstoff wurde nun festgestellt , daß bestimmte Derivate des Pyridoxins, insbesondere Pyridoxal, Pyridoxalphosphat, Pyridoxamin und Pyridoxaminphosphat und deren Vorläufer und Abbauprodukte überraschenderweise eine solche lipidsenkende, antiatherosklerotische Wirksamkeit besitzen. Dies wird in der DE 24 61 742 C2 ausdrücklich in Frage gestellt (Spalte 3, Zeile 35 ff). Das häufig auch als Vitamin $B_6$ bezeichnete Pyridoxin dagegen besitzt keine starke Wirkung in dieser Hinsicht. Pyridoxin ist in früheren tierexperimentellen Arbeiten vereinzelt mit dem Lipidstoffwechsel in Verbindung gebracht worden (vgl. Frolova,J.A.: Vitamin $B_6$ and lipid metabolism; Vopr.med.Khim. 89, 18. 339 - 346 (1972)). Die Wirkung ist aber schwach. In eigenen Untersuchungen hat sich Pyridoxin an Ratten bei einer Dosis von 79 mg/kg Körpergewicht als unwirksam erwiesen, obwohl in dem gleichen Experiment eine positive Kontrolle die erwartete Wirkung gezeigt hat.

Verschiedene Pyridoxin-Derivate treten im Stoffwechsel auf und sind teilweise wechselseitig ineinander überführbar. Dazu zählen neben dem Pyridoxin selbst beispielsweise Pyridoxamin, Pyridoxal, Pyridoxalphosphat und Pyridoxinsäure. Es ist allgemein anerkannt, daß durch orale Verabreichung von Pyridoxin der pool an Pyridoxal und dessen Derivaten entsprechend vermehrt wird.

Überraschenderweise hat sich nun herausgestellt, daß die orale Verabreichung bestimmter Pyridoxin-Derivate, insbesondere von Pyridoxalphosphat, Pyridoxal, Pyridoxaminphosphat und Pyridoxamin bzw. von deren Vorläufern jeweils zu einer deutlichen Verminderung der Serumlipide, insbesondere auch des für die Atherogenese so verhängnisvollen LDL-Cholesterins führt. Ein weiterer, von der Senkung der Serumlipide offenbar unabhängiger Effekt, ist die Verminderung der Einlagerung der Lipide in die Arterienwände. Beide Wirkungen garantieren eine effektive und nach heutigem Kenntnisstand an den Ursachen angreifende prophylaktische und therapeutische Behandlung der Atherosklerose. Eine solche Therapie ist umso wertvoller,als sie durch keine erkennbaren Nebenwirkungen belastet wird. Die Toxizität der genannten Vitamin $B_6$-Derivate ist außerordentlich niedrig. Bei den für die Therapie am Menschen in Frage kommenden Dosierungen ist daher eine sehr gute Verträglichkeit gegeben. Insbesondere tritt die lebertoxische Wirkung der meisten anderen Lipidsenker nicht auf, die sich bei den Versuchstieren schon nach kurzer Behand-

3

lungsdauer in einer ausgeprägten Steigerung der Lebergewichte äußert.

Die Erfindung betrifft daher die Verwendung von Pyridoxal und/oder Pyridoxamin oder Verbindungen von Pyridoxal und Pyridoxamin mit ihrerseits nicht lipidsenkenden Substanzen, sofern aus diesen Verbindungen in vitro und/oder in vivo Pyridoxal und/oder Pyridoxamin freigesetzt werden unter Ausnahme von solchen Derivaten, die gleichzeitig eine Phosphatgruppe und eine Aminosäure enthalten, zur Herstellung eines Arzneimittels für die Prophylaxe und Therapie von Hyperlipidämien und Arteriosklerose.

Die aufgefundene Wirksamkeit der genannten Pyridoxin-Derivate ist umso überraschender, da derart einfach aufgebaute Verbindungen, als wenig wirksam angesehen wurden und wirksame Arzneimittel, lediglich auf der Basis großer Komplexmoleküle des Pyridoxalphosphats erhofft wurden, wie beispielsweise in der bereits zitierten DE 24 61 742 C2 beschrieben ist.

Pyridoxamin- und Pyridoxal-p-chlorphenoxyisobutyrat gemäß DE-A-1 915 497 sind vom Schutzumfang ausgeschlossen, da der p-Chorphenoxyisobutyrat-Rest selbst eine lipidsenkende Wirkung aufweist.

Für die klinische Anwendung kommen erfindungsgemäß insbesondere die vier genannten Pyridoxin-Derivate (Pyridoxal, Pyridoxamin, Pyridoxalphosphat und Pyridoxaminphosphat) sowie beliebige Gemische derselben in Frage. Selbstverständlich sind auch alle Substanzen geeignet, welche die genannten Pyridoxin-Derivate in vitro und besonders auch in vivo freisetzen. Weiterhin ist es möglich, die genannten Pyridoxin-Derivate in Verbindung mit anderen, den Lipidstoffwechsel stabilisierenden Maßnahmen (z.B. Diät) oder Therapeutika (z.B. Gallensäureadsorbentien) einzusetzen. Kombinationen mit Arzneimitteln, die zur Therapie häufiger Begleiterkrankungen der Atherosklerose (Bluthochdruck, Diabetis etc,) geeignet sind, können sinnvoll sein. Wegen der sehr guten Verträglichkeit der genannten Pyridoxin- bzw. Vitamin $B_6$-Derivate bietet sich gerade der Einsatz bei Patienten mit vielfältigen Grund- oder Begleiterkrankungen an.

Nicht geeignet ist dagegen das Pyridoxin selbst gemäß FR-A-2 349 330, das sonst bevorzugt unter der Bezeichnung Vitamin $B_6$ eingesetzt wird.

Die Therapie geschieht in der Praxis bevorzugt durch orale Einnahme der betreffenden Wirkstoffe über längere Zeit. Ein maximaler Effekt ist erst nach zwei Monaten oder einer längeren Behandlungsdauer zu erwarten. Die Therapie ist kontinuierlich weiterzuführen, da es nach dem Absetzen der betreffenden Präparate sehr bald zu einem Abklingen auch der Wirkung, einem Wiederanstieg der Serumlipide und einer Progression der Atherosklerose kommt.

Im Ausnahmefall kann auch eine parenterale Verabreichung der erfindungsgemäß eingesetzten Wirkstoffe beispielsweise durch intravenöse Infusion der wässrigen Lösungen oder deren intramuskuläre Injektionen erfolgen.

Für die orale Therapie wird die Tagesdosis entweder insgesamt verabreicht oder auf 2 - 3 Teildosen verteilt.

Die Tagesdosis selbst kann zwischen 20 mg und 1000 mg liegen, vorzugsweise zwischen 50 und 500 mg der genannten Vitamin $B_6$-Derivate.

Die erfindungsgemäß eingesetzten Substanzen haben gegenüber den meisten anderen für die Atheroseprophylaxe und -therapie eingesetzten Arzneimitteln den wesentlichen Vorteil, geschmacklich neutral oder jedenfalls akzeptabel zu sein und durch leichte Wasserlöslichkeit auch von der Konsistenz her keinerlei Probleme zu verursachen. Der gute Geschmack und die gute Löslichkeit der erfindungsgemäßen Verbindungen erlauben die Nutzung der verbesserten Bioverfügbarkeit dieser Verbindungen im Anschluß an die buccale Absorption. Hier ist die Bioverfügbarkeit deutlich besser als beim Abbau im Magen-Darm-Bereich oder der Leber. Die sehr verbreiteten Clofibrate sind dagegen widerlich bitter, und die Gallensäureadsorbentien sind grobkörnig oder bilden voluminöse Gallerten.

Die erfindungsgemäß eingesetzten Substanzen können wegen ihrer Wasserlöslichkeit und chemischen Stabilität von älteren Patienten auch als Pulver, Granulat oder gelöst in Wasser oder Getränk leicht eingenommen werden. Ebenso ist es möglich, die Einzeldosis in Form einer Tablette, Kapsel oder eines Dragees anzubieten, wobei jede dieser Formen auch durch die dem Fachmann bekannten Maßnahmen in ihrer Auflösung bzw. der Freigabe des Wirkstoffes verzögert und gesteuert werden kann. Besonders hervorzuheben ist eine magensaftresistente Unmantelung, die den Wirkstoff erst im Darm freisetzt und vor der Magensäure schützt. Bei der Einnahme als Pulver, Granulat, Lösung oder auch Lutschtablette kann der Zusatz von Geschmacksstoffen zweckmäßig sein. Ansonsten können die Wirkstoffe in üblicher Weise als Salze, z.B. Pyridoxalhydrochlorid oder in Verbindung mit anderen physiologisch verträglichen Säuren oder Basen eingesetzt werden oder unter Verwendung von in der Pharmazie üblichen Hilfsstoffen, Stabilisatoren und Puffern zubereitet werden.

Die folgende Tabelle zeigt die Werte für das Absinken der Konzentration bestimmter Verbindungen bei hyperlipämischen Kaninchen nach 10-wöchiger Behandlung mit Pyridoxamin. Die Werte sind Mittelwerte von 18 Versuchstieren und relativ bezogen auf unbehandelte Kaninchen. Die Kontrollgruppe erhielt über 10 Wochen eine 2% Cholesterin enthaltene Diät, in der Versuchsgruppe erhielt jedes Tier über die selbe Zeit

EP 0 282 696 B1

zusätzlich 120 mg Pyridoxamin.2HCl/kg Körpergewicht. Anschließend wurden die Aorten der Tiere vom Herzen bis zum Abgang der Nierenarterien entnommen und untersucht:

| Trockengewicht | - 8 % |
|---|---|
| Gesamtlipide | - 18 % |
| Cholesterin | - 27 % |
| Triglyceride | - 17 % |
| Calcium | - 31 % |

Pyridoxamin.2HCl bewirkte folglich bei nur 10 wöchiger Behandlung eine deutliche Verminderung der für die Artherosklerose charakteristischen Veränderungen: Wandverdickung (erfaßt in Form des Trockengewichts), Lipid- und Calciumeinlagerung.

Beispiele

1. Kapseln

a) 500 g Pyridoxal .HCl oder Pyridoxamin.2 HCl werden mit 1000 g Lactose homogen vermischt und in Portionen zu je 150 mg in Gelatine-Steckkapseln gefüllt. Die Steckkapselhälften werden verklebt oder verschweißt. Die Einnahme je einer Kapsel erfolgt 3 x täglich zu den Mahlzeiten.
b) 300 mg Pyridoxal .HCl oder Pyridoxamin.2 HCl werden in Gelatinekapseln eingeschweißt. Die Einnahme erfolgt einmal täglich abends.

2. Tabletten zur buccalen Applikation

1000 g Pyridoxalphoshat-Magnesiumsalz werden mit 3000 g Lactose und 15 g Magnesiumstearat homogen vermischt, granuliert und zu Tabletten von je 301,5 mg verpreßt. Je eine Tablette wird morgens und abends langsam im Mund zergehen gelassen.

3. Magensaftresistente Tabletten

1000 g Pyridoxal.HCl oder Pyridoxamin.2 HCl werden mit 2000 g Tablettose (spezielle Lactose) und 15 g Magnesiumstearat homogen vermischt, granuliert und zu weitgehend runden Tabletten von 150 und 75 mg verpreßt. Anschließend werden 20 Überzüge aus Cellusoseacetat-succinat (9), Dimethylphthalat (3,4), Essigester (84,4) und Aceton (84,4) aufgebracht, wobei Talkum als Einstreumittel verwendet wird. 1 - 2 Tabletten werden 3 x täglich vor den Mahlzeiten eingenommen.

4. Brausetabletten

500 g Pyridoxalphosphat, 600 g Zitronensäure und 280 g Natriumkarbonat werden mit 2000 g Lactose vermischt und trocken zu Einheiten von je 338 mg verpreßt. Zu den Mahlzeiten werden 1 - 2 Tabletten in wenig Wasser gelöst eingenommen.

5. Granulat

1000 g Pyridoxal.HCl werden mit 1000 g Zitronensäure und 8000 g Lactose granuliert, und anschließend wird das Granulat gesiebt, um eine gleichmäßige Korngröße von ca. 1 mm zu erzielen. Das Granulat wird gut getrocknet und in Aluminiumfolie eingeschweißt. Die Einzeldosis je Beutel beträgt 500 mg oder 1 g. Der Beutelinhalt wird als Lösung in Wasser oder Fruchtsaft eingenommen. Zur Wirkungsverstärkung kann ein handelsübliches Guar-Präparat oder ein anderes lipidsenkendes Medikament, vorzugsweise ein Produkt, dessen Wirkstoff nicht resorbierbar ist oder dessen Wirkmechanismus eine Ergänzung darstellt, zugesetzt oder mit Hilfe der Lösung eingenommen werden.

**Patentansprüche**

**1.** Verwendung von Pyridoxal und/oder Pyridoxamin oder Verbindungen von Pyridoxal und Pyridoxamin mit ihrerseits nicht lipidsenkenden Substanzen, sofern aus diesen Verbindungen in vitro und/oder in

5

vivo Pyridoxal und/oder Pyridoxamin freigesetzt werden unter Ausnahme von solchen Derivaten, die gleichzeitig eine Phosphatgruppe und eine Aminosäure enthalten, zur Herstellung eines Arzneimittels für die Prophylaxe und Therapie von Hyperlipidämien und Arteriosklerose.

2. Verwendung von Pyridoxal und/oder Pyridoxalphosphat und/oder Pyridoxamin und/oder Pyridoxaminphosphat oder deren Salzen zur Herstellung eines Arzneimittels für die Prophylaxe und Therapie von Hyperlipidämien und Arteriosklerose.

3. Verwendung von Pyridoxin-Derivaten nach Anspruch 1, dadurch gekennzeichnet, daß das Pyridoxin-Derivat in fester Form oder als wäßrige Lösung zusammen mit pharmazeutisch unbedenklichen Träger- und/oder Hilfsstoffen hergestellt wird.

4. Verwendung von Pyridoxin-Derivaten nach mindestens einem der Ansprüche 1 bis 3, dadurch gekennzeichnet, daß sie mit üblichen Trägern und Hilfsstoffen zur buccalen Application hergestellt werden.

5. Verwendung von Pyridoxin-Derivaten nach mindestens einem der Ansprüche 1 bis 3, dadurch gekennzeichnet, daß sie mit üblichen Trägern und Hilfsmitteln in einer magensaftresistenten Form hergestellt werden.

## Claims

1. Use of pyridoxal and/or pyridoxamine or compounds of pyridoxal and pyridoxamine with substances which are not for their part lipid-lowering as long as pyridoxal and/or pyridoxamine are released from these compounds in vitro and/or in vivo, with the exception of those derivatives which simultaneously contain a phosphate group and an amino acid, for the production of a pharmaceutical for the prophylaxis and therapy of hyperlipidaemias and arteriosclerosis.

2. Use of pyridoxal and/or pyridoxal phosphate and/or pyridoxamine and/or pyridoxamine phosphate or the salts thereof for the production of a pharmaceutical for the prophylaxis and therapy of hyperlipidaemias and arteriosclerosis.

3. Use of pyridoxine derivatives according to Claim 1, characterised in that the pyridoxine derivative is prepared in solid form or as aqueous solution together with pharmaceutically acceptable vehicles and/or ancillary substances.

4. Use of pyridoxine derivatives according to at least one of Claims 1 to 3, characterised in that they are prepared with conventional vehicles and ancillary substances for buccal administration.

5. Use of pyridoxine derivatives according to at least one of Claims 1 to 3, characterised in that they are prepared with conventional vehicles and aids in a form which is resistant to gastric fluid.

## Revendications

1. Utilisation de pyridoxal et/ou de pyridoxamine, ou de composés du pyridoxal et de la pyridoxamine avec des substances non hypolipidémiantes en ce qui les concerne, dans la mesure où ces composés libèrent du pyridoxal et/ou de la pyridoxamine in vivo et/ou in vitro, à l'exception des dérivés qui contiennent à la fois un groupe phosphate et un amino-acide, pour la préparation d'un médicament destiné à la prophylaxie et à la thérapie de l'hyperlipidémie et de l'artériosclérose.

2. Utilisation de pyridoxal et/ou de phosphate de pyridoxal et/ou de pyridoxamine et/ou de phosphate de pyridoxamine ou de sels de ceux-ci, pour la préparation d'un médicament destiné à la prophylaxie et à la thérapie de l'hyperlipidémie et de l'artériosclérose.

3. Utilisation de dérivés de pyridoxine selon la revendication 1, caractérisée en ce qu'on prépare le dérivé de pyridoxine sous forme solide ou sous forme de solution aqueuse, conjointement avec des véhicules et/ou des adjuvants pharmacologiquement acceptables.

4. Utilisation de dérivés de pyridoxine selon au moins une des revendications 1 à 3, caractérisée en ce qu'on prépare ceux-ci avec des véhicules et des adjuvants courants pour l'administration orale.

5. Utilisation de dérivés de pyridoxine selon au moins une des revendications 1 à 3, caractérisée en ce qu'on prépare ceux-ci avec des véhicules et des adjuvants, sous une forme résistant aux sucs gastriques.